(19) **Europäisches Patentamt / European Patent Office / Office européen des brevets**

(11) **EP 4 166 639 A1**

(12) **EUROPEAN PATENT APPLICATION**
published in accordance with Art. 153(4) EPC

(43) Date of publication:
**19.04.2023 Bulletin 2023/16**

(21) Application number: **21822557.1**

(22) Date of filing: **26.01.2021**

(51) International Patent Classification (IPC):
| | |
|---|---|
| C11D 17/08 (2006.01) | A61Q 11/00 (2006.01) |
| A61Q 11/02 (2006.01) | C11D 1/04 (2006.01) |
| C11D 1/10 (2006.01) | C11D 1/14 (2006.01) |
| C11D 1/18 (2006.01) | C11D 1/68 (2006.01) |
| C11D 1/74 (2006.01) | C11D 1/75 (2006.01) |
| C11D 1/90 (2006.01) | C11D 1/92 (2006.01) |
| C11D 3/37 (2006.01) | A61K 8/36 (2006.01) |
| A61K 8/37 (2006.01) | A61K 8/41 (2006.01) |
| A61K 8/44 (2006.01) | A61K 8/46 (2006.01) |
| A61K 8/49 (2006.01) | A61K 8/60 (2006.01) |

(52) Cooperative Patent Classification (CPC):
**A61K 8/36; A61K 8/37; A61K 8/40; A61K 8/41;
A61K 8/44; A61K 8/46; A61K 8/49; A61K 8/60;
A61K 8/86; A61Q 11/00; A61Q 11/02; C11D 1/04;
C11D 1/10; C11D 1/14; C11D 1/18;** (Cont.)

(86) International application number:
**PCT/JP2021/002626**

(87) International publication number:
**WO 2021/250926 (16.12.2021 Gazette 2021/50)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **11.06.2020 JP 2020101886
24.08.2020 JP 2020141085**

(71) Applicant: **Kao Corporation
Chuo-ku
Tokyo 103-8210 (JP)**

(72) Inventors:
- **SONOI, Atsunori
Tokyo 131-8501 (JP)**
- **HANDA, Takuya
Tokyo 131-8501 (JP)**
- **SATOU, Tomoya
Tokyo 131-8501 (JP)**

(74) Representative: **Hoffmann Eitle
Patent- und Rechtsanwälte PartmbB
Arabellastraße 30
81925 München (DE)**

(54) **CLEANING AGENT COMPOSITION FOR INTRAORALLY MOUNTED APPLIANCES**

(57) The present invention relates to a cleaning composition for an oral appliance which has high cleaning performance and can exert an excellent denture plaque removal effect and formation suppressing effect. Specifically, the present invention provides a cleaning composition for an oral appliance, comprising the following components (A) and (B): (A) a fatty acid having 10 or more and 20 or less carbon atoms or a salt thereof in an amount of 0.5 mass% or more and 20 mass% or less in terms of the fatty acid; and (B) one or more surfactants selected from the group consisting of an anionic surfactant (b1), a nonionic surfactant (b2), and an amphoteric surfactant (b3), wherein the composition a pH at 25°C of 5.5 or higher and 14 or lower.

EP 4 166 639 A1

(52) Cooperative Patent Classification (CPC): (Cont.)
**C11D 1/74; C11D 1/75; C11D 1/90; C11D 1/92;**
**C11D 3/2003; C11D 3/37; C11D 17/08**

**Description**

Field of the Invention

The present invention relates to a cleaning composition for an oral appliance.

Background of the Invention

As the population has super-aged in recent years, the number of people obliged to wear an oral appliance such as dentures is increasing at an accelerated rate. In such an oral appliance, dirt such as a denture plaque or food residues, attached during wear tends to easily remain. Hence, the oral appliance easily forms a nest for a denture plaque even after removal of the oral appliance. As a result, if a person re-wears the oral appliance as it is, bad breaths, dental caries, gum diseases, or the like may be caused. Furthermore, the oral appliance becomes slimy and may cause unpleasant feeling during wear.

Thus, oral appliances need to be kept clean and inevitably require daily elaborate care, also for maintaining physical health.

Under these circumstances, various compositions have been developed in order to contribute to the accomplishment of care of dentures. For example, Patent Literature 1 discloses a coating-type liquid denture cleanser composition in which a nonionic surfactant, a cationic fungicide, sulfite and a water soluble polymer material are blended at specific amounts and specific mass ratios, and which has a specific value of pH. This composition enhances a stain dirt removal effect on dentures or the antiseptic force of a dipping solution upon denture dipping. Patent Literature 2 discloses an antimicrobial agent for a denture containing 15 to 45 mass% of a fatty acid having 8 to 22 carbon atoms or a salt thereof, specific fatty acid glyceride, and a specific amount of sulfosuccinic acid ester or the like. This agent is effective for the sterilization or disinfection of bacteria and fungi.

Patent Literatures

(Patent Literature 1) JP-A-2010-280588
(Patent Literature 2) JP-A-2011-195496

Summary of the Invention

The present invention provides a cleaning composition for an oral appliance, comprising the following components (A) and (B):

(A) a fatty acid having 10 or more and 20 or less carbon atoms or a salt thereof in an amount of 0.5 mass% or more and 20 mass% or less in terms of the fatty acid; and
(B) one or more surfactants selected from the group consisting of an anionic surfactant (b1), a nonionic surfactant (b2), and an amphoteric surfactant (b3), wherein

the component (b1) is one or more selected from the group consisting of an N-acyl amino acid, N-acyl taurine, and an α-olefinsulfonic acid, and salts thereof,
the component (b2) is one or more selected from the group consisting of polyoxyethylene hydrogenated castor oil, sorbitan fatty acid ester, fatty acid polyoxyethylene sorbitan ester, sucrose fatty acid ester, and alkyl glycoside,
the component (b3) is one or more selected from the group consisting of betaine acetate, cocamidoalkyl betaine, alkyl sulfobetaine, imidazolinium betaine, carboxybetaine, alkylimidazoline betaine salt, and alkylamine oxide, and
the composition has a pH at 25°C of 5.5 or higher and 14 or lower.

The liquid composition for denture cleaning described in Patent Literature 1 requires application over a long time using a dipping solution or a dissolving solution, or physical additive force such as brushing, for cleaning dentures, and this situation imposes burdens on users. Also, the antimicrobial agent for a denture described in Patent Literature 2 cannot sufficiently remove a denture plaque attached firmly to a removed oral appliance. In this situation, the agent is still susceptible to improvement in order to achieve better cleaning of an oral appliance. Furthermore, no study has been made on low-temperature storage stability.

Specifically, the present invention relates to a cleaning composition for an oral appliance which has high cleaning performance and can exert an excellent denture plaque removal effect and formation suppressing effect.

Accordingly, the present inventors conducted diligent studies to solve these problems and consequently found a cleaning composition for an oral appliance, comprising a very limited amount of a specific fatty acid or salt thereof and a specific surfactant, and having pH in a specific range, whereby the cleaning composition not only can remove a denture plaque firmly attached to an oral appliance such as dentures and suppress the formation thereof, but also is excellent in low-temperature storage stability.

The cleaning composition for an oral appliance of the present invention can exert a highly fast-acting, excellent denture plaque removal effect and formation suppressing effect, in addition, is also excellent in low-temperature storage stability, and can validly suppress the solidification of the composition, the precipitation of a contained component, and the generation of unpleasant odor or the like.

Detailed Description of the Invention

Hereinafter, the present invention will be described in detail.

In the present specification, the "oral appliance" means a so-called dental appliance which needs to be attached and removed in the oral cavity, such as complete dentures, partial dentures, or an orthodontic appliance (hereinafter, these are also collectively referred to as "dentures or the like"), or a mouthpiece or a mouthguard. Specifically, for example, the complete dentures (complete false teeth) are dentures to be mounted in the edentulous jaw and are constituted by artificial teeth and a denture base. On the other hand, the partial dentures (partial false teeth) are dentures to be mounted in the jaw partially lacking teeth and are constituted by artificial teeth, a denture base, and an anchor device such as a clasp, a rest, or an attachment. The orthodontic appliance or the retainer has a constitution similar thereto. Examples of the material of the denture base generally include resins such as acrylic resin. Examples of the material of the artificial teeth or the anchor device include such resins as well as metals such as titanium, and ceramic. Examples of the material of the mouthpiece or the mouthguard generally include resins such as ethylene vinyl acetate.

In the present specification, the "denture plaque" means an aggregate of bacteria attached to an oral appliance, and extracellular substances produced by the bacteria.

The cleaning composition for an oral appliance of the present invention contains, as a component (A), a fatty acid having 10 or more and 20 or less carbon atoms or salt thereof (A) in an amount of 0.5 mass% or more and 20 mass% or less in terms of the fatty acid. This component (A) is strongly adsorbed onto the surface of a denture plaque firmly attached to an oral appliance removed from the oral cavity. Thus, the component (A) can destroy the aggregation structure of the denture plaque and effectively eradicate the denture plaque from the oral appliance. Therefore, the component (A) is considered to exert an excellent denture plaque removal effect and enhance cleaning performance.

The fatty acid as the component (A) or the fatty acid constituting the fatty acid salt as the component (A) has 10 or more and 20 or less, preferably 12 or more and 20 or less, more preferably 14 or more and 20 or less, further more preferably 16 or more and 20 or less carbon atoms. The fatty acid optionally has a saturated or unsaturated linear or branched chain and more preferably has an unsaturated linear chain.

Specific examples of the fatty acid as the component (A) or the fatty acid constituting the fatty acid salt as the component (A) include one or more selected from the group consisting of capric acid, lauric acid, myristic acid, palmitic acid, stearic acid, palmitoleic acid, oleic acid, linoleic acid, linolenic acid, vaccenic acid, gadoleic acid, eicosenoic acid, isostearic acid, and arachidic acid, and coconut oil fatty acid and palm oil fatty acid which are mixed fatty acids thereof. Among them, palmitoleic acid, oleic acid, linoleic acid, linolenic acid, vaccenic acid, gadoleic acid, and/or eicosenoic acid is preferred, and oleic acid is more preferred, from the viewpoint of sufficiently exerting a denture plaque removal effect.

Examples of the salt of the component (A) include one or more selected from the group consisting of: inorganic salt selected from the group consisting of sodium salt, potassium salt, magnesium salt, and ammonium salt; organic amine salt such as monoethanol ammonium salt, diethanol ammonium salt, and triethanol ammonium salt; and basic amino acid salt such as alginate. Among them, sodium salt or potassium salt is preferred, and potassium salt is particularly preferred, from the viewpoint of sufficiently exerting a denture plaque removal effect.

The content of the component (A) in the cleaning composition for an oral appliance of the present invention is 0.5 mass% or more, preferably 0.6 mass% or more, more preferably 0.7 mass% or more, further more preferably 0.8 mass% or more, in terms the fatty acid, from the viewpoint of validly exerting an excellent denture plaque removal effect. The content of the component (A) in the cleaning composition for an oral appliance of the present invention is 20 mass% or less, preferably 14 mass% or less, more preferably 5 mass% or less, further more preferably 3 mass% or less, in terms of the fatty acid, from the viewpoint of preventing unpleasant flavor from being generated during wear of an oral appliance after application, while securing the strong adsorbability of the component (A) to denture plaque surface. The content of the component (A) in the cleaning composition for an oral appliance of the present invention is from 0.5 mass% to 20 mass%, preferably from 0.6 to 14 mass%, more preferably from 0.7 to 5 mass%, further more preferably from 0.8 to 3 mass%, in terms of the fatty acid.

The cleaning composition for an oral appliance of the present invention contains, as a component (B), one or more surfactants selected from the group consisting of a specific anionic surfactant (b1), a specific nonionic surfactant (b2), and a specific amphoteric surfactant (b3). This component (B), which is a specific surfactant, contained together with the component (A) is capable of exerting a denture plaque formation suppressing effect and enhancing the low-temperature storage stability of the component (A), and can allow the component (A) to validly exert an excellent denture plaque removal effect. Furthermore, unpleasant odor of fatty acid derived from the component (A) can also be effectively suppressed.

The anionic surfactant as the component (b1) is one or more selected from the group consisting of an N-acyl amino acid, N-acyl taurine and an α-olefinsulfonic acid, and salts thereof. This component (b1), which is an anionic surfactant having a carboxylic acid group, contained together with the component (A) is capable of exerting a denture plaque formation suppressing effect and enhancing the low-temperature storage stability of the component (A), and can allow the component (A) to validly exert an excellent denture plaque removal effect. Furthermore, unpleasant odor of fatty acid derived from the component (A) can also be effectively suppressed.

The acyl group of the N-acyl amino acid or the N-acyl taurine is derived from a fatty acid having a saturated or unsaturated linear or branched chain or a mixed fatty acid thereof, preferably a linear fatty acid or a mixed fatty acid of linear fatty acids, and is preferably an acyl group having 6 to 22 carbon atoms, more preferably an acyl group having 10 to 20 carbon atoms, further more preferably an acyl group having 12 to 18 carbon atoms, from the viewpoint of bringing about excellent low-temperature storage stability while allowing the component (A) to validly exert an excellent denture plaque removal effect.

The acyl group is preferably one or more selected from the group consisting of a capryloyl group, a lauroyl group, a myristoyl group, a palmitoyl group, a stearoyl group, and a cocoyl group, more preferably one or more selected from the group consisting of a lauroyl group, a myristoyl group, and a cocoyl group, further more preferably one or more selected from the group consisting of a lauroyl group and a myristoyl group, further more preferably a lauroyl group, from the viewpoint of the low-temperature storage stability of the cleaning composition for an oral appliance. When the acyl group is a cocoyl group, unpleasant odor of fatty acid derived from the component (A) can also be effectively suppressed.

The amino acid moiety constituting the N-acyl amino acid may be any of glutamic acid, aspartic acid, and glycine and may be any of a D form, an L form, and a mixture of D and L forms, and is preferably an L form. Specifically, the N-acyl amino acid is preferably one or more selected from the group consisting of N-lauroyl glutamic acid, N-myristoyl glutamic acid, N-cocoyl glutamic acid, N-lauroyl aspartic acid, N-myristoyl aspartic acid, N-cocoyl aspartic acid, N-lauroyl glycine, N-myristoyl glycine, and N-cocoyl glycine, more preferably one or more selected from the group consisting of N-lauroyl glutamic acid and N-myristoyl glutamic acid. When the N-acyl amino acid is N-cocoyl glutamic acid, unpleasant odor of fatty acid derived from the component (A) can also be effectively suppressed.

The N-acyl taurine is preferably one or more selected from the group consisting of cocoyl fatty acid taurine, cocoyl methyl taurine, N-caproyl methyl taurine, N-lauroyl taurine, N-lauroyl methyl taurine, N-myristoyl methyl taurine, N-palmitoyl methyl taurine, N-stearoyl methyl taurine, and N-oleoyl methyl taurine, more preferably one or more selected from the group consisting of N-lauroyl methyl taurine and N-myristoyl methyl taurine. N-Lauroyl methyl taurine is preferred.

The olefin group of the α-olefinsulfonic acid or the salt thereof preferably has 8 to 18 carbon atoms, more preferably 14 to 16 carbon atoms, further more preferably 14 carbon atoms.

Examples of salts thereof include: salts of alkali metals such as sodium and potassium; salts of alkaline earth metals such as calcium and magnesium; other inorganic salts of aluminum, zinc, and the like; ammonium salt; salts of organic amine such as monoethanolamine, diethanolamine, and triethanolamine; and basic amino acid salt such as arginine salt, lysine salt, histidine salt, and ornithine salt. These salts may each be used singly or may be used in combination of two or more thereof. Among them, alkali metal salt is preferred, and sodium salt and/or potassium salt is more preferred, from the viewpoint of the suppression of unpleasant odor of fatty acid derived from the component (A), and easy availability.

The nonionic surfactant as the component (b2) is one or more selected from the group consisting of polyoxyethylene hydrogenated castor oil, sorbitan fatty acid ester, fatty acid polyoxyethylene sorbitan ester, sucrose fatty acid ester, and alkyl glycoside.

The average number of moles of the ethyleneoxy group added in the polyoxyethylene hydrogenated castor oil is preferably from 20 to 100 mol, more preferably from 40 to 80 mol.

The amphoteric surfactant as the component (b3) is one or more selected from the group consisting of: betaine acetate such as lauryl betaine dimethylaminoacetate; cocamidoalkyl betaine such as cocamidopropyl betaine; alkyl sulfobetaine such as lauryl sulfobetaine and lauryl hydroxy sulfobetaine; imidazolinium betaine such as 2-alkyl-N-carboxymethyl-N-hydroxyethyl-N-imidazolium betaine; carboxybetaine; alkylimidazoline betaine salt such as sodium N-alkyl-1-hydroxyethylimidazoline betaine; and alkylamine oxide such as lauryl dimethylamine oxide.

Among them, one or more selected from the group consisting of cocamidoalkyl betaine, alkyl sulfobetaine, and alkylamine oxide is preferred, cocamidoalkylpropyl betaine and/or alkylamine oxide is more preferred, and alkylamine oxide is further more preferred.

Among these components (B), the component (b1) is preferred, and one or more selected from the group consisting of an N-acyl amino acid and N-acyl taurine and salts thereof is more preferred, from the viewpoint of validly exerting both a denture plaque formation suppressing effect and denture plaque removal effect and from the viewpoint of enhancing the low-temperature storage stability of the component (A).

The cleaning composition for an oral appliance of the present invention preferably contains the component (b1) and also contains alkyl sulfate from the viewpoint of further enhancing low-temperature storage stability. The alkyl sulfate preferably has a saturated or unsaturated alkyl group having 8 to 18 carbon atoms and more preferably has a saturated alkyl group having 10 to 12 carbon atoms.

In the case of containing the alkyl sulfate, the content thereof is preferably 0.01 mass% or more, more preferably 0.03 mass% or more, further more preferably 0.05 mass% or more, even more preferably 0.07 mass% or more, and preferably 8 mass% or less, more preferably 4 mass% or less, further more preferably 2 mass% or less, even more preferably 1 mass% or less, in the cleaning composition for an oral appliance of the present invention.

The content of the component (B) is preferably 0.05 mass% or more, more preferably 0.07 mass% or more, further more preferably 0.15 mass% or more, even more preferably 0.25 mass% or more, even more preferably 0.5 mass% or more, in the cleaning composition for an oral appliance of the present invention from the viewpoint of effectively enhancing low-temperature storage stability and the suppression of unpleasant odor of fatty acid derived from the component (A) while validly exerting an excellent denture plaque removal effect. The content of the component (B) is preferably 5 mass% or less, more preferably 4 mass% or less, further more preferably 3 mass% or less, in the cleaning composition for an oral appliance of the present invention from the viewpoint of solubility in water. The content of the component (B) is preferably from 0.05 mass% to 5 mass%, more preferably from 0.07 to 4 mass%, further more preferably from 0.15 to 3 mass%, even more preferably from 0.25 to 3 mass%, even more preferably from 0.5 to 3 mass%, in the cleaning composition for an oral appliance of the present invention.

The pH at 25°C of the cleaning composition for an oral appliance of the present invention is 5.5 or higher, preferably 6 or higher, more preferably 6.5 or higher, further more preferably 7 or higher, even more preferably 7.5 or higher, even more preferably 8 or higher, even more preferably 8.5 or higher, even more preferably 8.8 or higher, even more preferably 9 or higher, even more preferably 9.5 or higher, and 14 or lower, preferably 13 or lower, more preferably 12 or lower, further more preferably 11 or lower, from the viewpoint of validly enhancing a denture plaque removal effect and appearance immediately after production.

The mass ratio of the content of the component (B) to the content of the component (A), ((B)/(A)), is preferably 0.05 or more, more preferably 0.15 or more, further more preferably 0.25 or more, even more preferably 0.35 or more, even more preferably 0.5 or more, from the viewpoint of rapidly eliminating a denture plaque from an oral appliance, improving low-temperature storage stability, and suppressing unpleasant odor of fatty acid derived from the component (A). The mass ratio of the content of the component (B) to the content of the component (A), ((B)/(A)), is preferably 5 or less, preferably 2.5 or less, more preferably 2.2 or less, further more preferably 2 or less, from the viewpoint of validly exerting an excellent denture plaque formation suppressing effect. The mass ratio of the content of the component (B) to the content of the component (A), ((B)/(A)), is preferably 0.05 or more and 5 or less, more preferably from 0.15 to 2.5, further more preferably from 0.25 to 2.2, even more preferably from 0.35 to 2, even more preferably from 0.5 to 2.

When the component (B) is one or more selected from the group consisting of α-olefinsulfonic acid and alkylamidopropyl betaine, the mass ratio of the content of the component (B) to the content of the component (A), ((B)/(A)), is preferably from 0.05 to 10, more preferably from 0.15 to 8, further more preferably from 0.25 to 7, even more preferably from 0.35 to 6, even more preferably from 0.5 to 5, from the viewpoint of rapidly eliminating a denture plaque from an oral appliance, improving low-temperature storage stability

The cleaning composition for an oral appliance of the present invention preferably further contains an anionic homopolymer or copolymer (C) from the viewpoint of exerting a better denture plaque removal effect and further enhancing cleaning performance. The anionic homopolymer or copolymer is an anionic homopolymer or copolymer comprising a constituent unit derived from an anionic monomer. When the component (A) is used, the component (C) contained together with the component (B) can allow the component (A) to validly exert an excellent denture plaque removal effect.

The component (C) may be a homopolymer composed of a constituent unit derived from only one anionic monomer, may be a copolymer composed of constituent units derived from two or more anionic monomers, or may be a copolymer composed of a constituent unit(s) derived from anionic monomer(s) and a constituent unit(s) derived from an additional monomer(s) other than the anionic monomer.

The mass average molecular weight of the component (C) is preferably 2,000 or higher, more preferably 5,000 or higher, further more preferably 8,000 or higher, even more preferably 10,000 or higher, from the viewpoint of validly exerting a denture plaque removal effect. The mass average molecular weight of the component (C) is preferably 500,000 or lower, more preferably 350,000 or lower, further more preferably 200,000 or lower, even more preferably 100,000 or lower, from the viewpoint of securing favorable solubility or dispersibility of each component. The mass average molecular weight of the component (C) is preferably from 2,000 to 500,000, more preferably from 5,000 to 350,000, further more preferably from 8,000 to 200,000, even more preferably from 10,000 to 100,000.

The mass average molecular weight (Mw) means a value determined by gel permeation chromatography (which uses a chloroform solvent, a calibration curve defined with linear polystyrene as a standard, and a refractive index detector) measurement.

The anionic monomer-derived constituent unit contained in the component (C) is preferably a constituent unit derived from (c1) an anionic monomer having a carboxyl group, a sulfo group, or a phosphoric acid group, from the viewpoint of making a major contribution to the exertion of a denture plaque removal effect. Specific examples of the monomer (c1) constituting the component (C) include one or more selected from the group consisting of acrylic acid, methacrylic acid, itaconic acid, maleic acid, fumaric acid, sulfonic acid, styrenesulfonic acid, methacryloyloxyalkylsulfonic acid, methacrylamidoalkylsulfonic acid, acryloyloxyalkyl phosphate, and their anhydrides, their monomers partially substituted by an alkyl group or the like, and monomers derived from sugars such as glucopyranose in which carboxymethyl groups are bonded to some hydroxy groups. Among them, a monomer containing no aromatic or alicyclic ring in the molecular structure is preferred. One or more selected from the group consisting of acrylic acid, methacrylic acid, maleic acid, and sulfonic acid is more preferred.

The content of the constituent unit derived from the anionic monomer in 100 mass% in total of constituent units of the component (C) is preferably 15 mass% or more, more preferably 35 mass% or more, further more preferably 50 mass% or more, and preferably 100 mass% or less, from the viewpoint of validly securing the exertion of an excellent denture plaque removal effect.

Specific examples of the component (C) include one or more selected from the group consisting of an acrylic acid homopolymer, a methacrylic acid homopolymer, a cross-linked acrylic acid homopolymer, an acrylic acid/maleic acid copolymer, an acrylic acid/methacrylic acid copolymer, an acrylic acid/sulfonic acid copolymer, a vinyl acetate/maleic acid copolymer, a phosphinic acid/acrylic acid copolymer, carboxymethylcellulose, xanthan gum, gellan gum, alginic acid, carrageenan, pectin, and hyaluronic acid. Among them, one or more selected from the group consisting of an acrylic acid homopolymer, an acrylic acid/maleic acid copolymer, an acrylic acid/methacrylic acid copolymer, and an acrylic acid/sulfonic acid copolymer is preferred, one or more selected from the group consisting of an acrylic acid/maleic acid copolymer and an acrylic acid/sulfonic acid copolymer is more preferred, and an acrylic acid/maleic acid copolymer is further preferred, from the viewpoint of exerting an excellent denture plaque removal effect while validly exerting even a denture plaque formation suppressing effect.

The mass ratio of a constituent unit derived from an acrylic acid monomer and a constituent unit derived from a maleic acid monomer, constituting the acrylic acid/maleic acid copolymer, (acrylic acid/maleic acid) is preferably from 0.01 to 99, more preferably from 0.05 to 50, further more preferably from 0.1 to 10, even more preferably 0.1 to 5.

The content of the component (C) is preferably 0.05 mass% or more, more preferably 0.07 mass% or more, further more preferably 0.2 mass% or more, even more preferably 0.3 mass% or more, in the cleaning composition for an oral appliance of the present invention from the viewpoint of exerting an excellent denture plaque removal effect. The content of the component (C) is preferably 8 mass% or less, more preferably 7 mass% or less, further more preferably 6 mass% or less, even more preferably 5 mass% or less, even more preferably 4 mass% or less, even more preferably 2 mass% or less, in the cleaning composition for an oral appliance of the present invention from the viewpoint of securing favorable solubility or dispersibility of each component and from the viewpoint of ensuring low-temperature storage stability. The content of the component (C) is preferably from 0.05 mass% to 8 mass%, more preferably from 0.07 to 7 mass%, further more preferably from 0.2 to 6 mass%, even more preferably from 0.2 to 5 mass%, even more preferably from 0.3 to 4 mass%, even more preferably from 0.3 to 2 mass%, in the cleaning composition for an oral appliance of the present invention.

The mass ratio of the content of the component (C) to the content of the component (A), ((C)/(A)), is preferably from 0.05 to 8, more preferably from 0.07 to 7, further more preferably from 0.1 to 6, even more preferably from 0.2 to 5, even more preferably from 0.3 to 4, even more preferably from 0.3 to 2, from the viewpoint of exerting an excellent denture plaque removal effect and rapidly eliminating a denture plaque from an oral appliance.

The cleaning composition for an oral appliance of the present invention preferably further contains water (D) from the viewpoint of promoting the exertion of a denture plaque removal effect by the component (A) and also enhancing the diffusivity of the cleaning composition for an oral appliance after its application to an oral appliance, while favorably dispersing or dissolving each component.

The content of the component (D) is preferably more than 50 mass%, more preferably 56 mass% or more, further more preferably 60 mass% or more, and preferably 99 mass% or less, more preferably 98.5 mass% or less, further more preferably 98 mass% or less, in the cleaning composition for an oral appliance of the present invention.

The water according to the present invention means the whole moisture contained in the cleaning composition for an oral appliance, including not only purified water or the like directly blended into the cleaning composition for an oral appliance, but also moisture contained in each component blended. The water according to the present invention is meant to include water blended in advance into the cleaning composition for an oral appliance, and if this is not the case, for example, in the state diluted with water or the like or dissolved in water or the like before use, meant to also include water used in the dilution or dissolution.

The mass ratio of the content of the component (D) to the content of the component (A), ((D)/(A)), is preferably from 50 to 99, more preferably from 55 to 98.5, further more preferably from 60 to 98, from the viewpoint of rapidly eliminating a denture plaque from an oral appliance and improving low-temperature storage stability.

The cleaning composition for an oral appliance of the present invention can further contain a pH adjuster (E). This can maintain pH effective for enhancing a denture plaque removal effect and appearance immediately after production.

Specific examples of the component (E) include one or more selected from the group consisting of: carbonate and bicarbonate; hydroxide; organic acids such as citric acid, malic acid, lactic acid, tartaric acid, and succinic acid, and their salts. Among them, one or more selected from the group consisting of carbonate, bicarbonate, and hydroxide is preferred, one or more selected from the group consisting of sodium carbonate, sodium bicarbonate, sodium sesquicarbonate, sodium hydroxide, potassium carbonate, potassium bicarbonate, potassium sesquicarbonate and potassium hydroxide is more preferred, and one or more selected from the group consisting of potassium carbonate, potassium bicarbonate, potassium sesquicarbonate, and potassium hydroxide is further more preferred. In this case, the content of the pH adjuster is preferably from 0.1 to 60 mass%, more preferably from 0.1 to 20 mass%, further more preferably from 0.15 to 10 mass%, even more preferably from 0.2 to 5 mass%, in the cleaning composition for an oral appliance of the present invention from the viewpoint of stabilizing the pH of the cleaning composition for an oral appliance of the present invention.

The mass ratio of the content of the component (E) to the content of the component (A), ((E)/(A)), is preferably from 0.1 to 60, more preferably from 0.1 to 20, further more preferably from 0.15 to 10, even more preferably from 0.2 to 5, even more preferably from 0.2 to 2, from the viewpoint of rapidly eliminating a denture plaque from an oral appliance and improving low-temperature storage stability.

The cleaning composition for an oral appliance of the present invention can contain, for example, a surfactant other than the components described above, an anti-inflammatory agent, an antiseptic, a fragrance, a pigment, a dye, a brightener, or an enzyme in addition to the components described above, without inhibiting the advantageous effects of the present invention.

The form of the cleaning composition for an oral appliance of the present invention may be a liquid, foam, paste, gel, concentrated, powder, granule, or tablet form, and may be the form of a sheet impregnated with the cleaning composition for an oral appliance of the present invention in a liquid state, the form of a spray container filled with the cleaning composition for an oral appliance of the present invention, the form of a foam discharge container filled therewith, or the form of an aerosol container filled therewith.

In the case of applying the cleaning composition for an oral appliance of the present invention to a concentrated, powder, granule, or tablet form, the low-temperature stability of the cleaning composition for an oral appliance of the present invention means the low-temperature stability of the composition diluted with water or the like or dissolved in water or the like.

Among them, the cleaning composition for an oral appliance of the present invention is preferably in the form of a foam, and can effectively exert an excellent denture plaque removal effect and formation suppressing effect by covering an oral appliance with the foam.

In particular, the form of a foam allows the cleaning composition to sufficiently reach every corner of an oral appliance having a complicated shape by covering the oral appliance with the foam, and can validly and effectively exert an excellent denture plaque removal effect and formation suppressing effect. As a result, a highly fast-acting, excellent denture plaque removal effect and formation suppressing effect can be exerted, without particular physical additive force such as brushing, by merely rinsing the oral appliance after application of the composition to wash off the composition.

The usage pattern of the cleaning composition for an oral appliance of the present invention involves first dipping an oral appliance such as dentures in the cleaning composition for an oral appliance or applying the cleaning composition for an oral appliance to the oral appliance by foam discharge, application, dropwise addition or spraying, or the like, and then leaving them for a given time. The leaving time can usually be from 20 to 30 minutes. The leaving time may be shortened to from 5 to 10 minutes.

Subsequently, the oral appliance thus left is rinsed with water or the like to wash off the cleaning composition for an oral appliance. More preferably, the cleaning composition for an oral appliance of the present invention is just washed off and doesn't require particular physical additive force such as brushing.

When the cleaning composition for an oral appliance of the present invention is in the form of a foam, the usage pattern thereof involves covering an oral appliance such as dentures with the cleaning composition for an oral appliance in a foam, and then leaving them for a given time.

An amount of time for which the oral appliance is covered with the foam is preferably 1 minute or longer, more preferably 2 minutes or longer, further more preferably 3 minutes or longer, from the viewpoint of sufficiently attaining an excellent denture plaque removal effect and formation suppressing effect. An amount of time for which the oral appliance is covered with the foam is preferably 30 minutes or shorter, more preferably 20 minutes or shorter, further more preferably 10 minutes or shorter, from the viewpoint of sufficiently exerting the effect of the foam. An amount of time for which the oral appliance is covered with the foam is preferably from 1 minute to 30 minutes, more preferably from 2 to 20 minutes, further more preferably from 3 to 10 minutes.

The volume of the foam used to cover the oral appliance is preferably 0.05 g or more, more preferably 0.07 g or more, further more preferably 0.1 g or more, and preferably 3.5 g or less, more preferably 3.2 g or less, further more preferably 3 g or less, per cm$^2$ of the surface area of the oral appliance from the viewpoint of sufficiently exerting the effect of the foam and sufficiently attaining an excellent denture plaque removal effect and formation suppressing effect.

After the oral appliance is covered with the formed foam, the cleaning composition is preferably washed off by rinsing the oral appliance with water. Thus, the cleaning composition for an oral appliance of the present invention in the form of a foam can exert a highly fast-acting, excellent denture plaque removal effect and formation suppressing effect while the covering foam is washed off by merely rinsing the oral appliance with water without the need of particular physical additive force. Therefore, this composition can also reduce burdens on users such as elderly people and facilitate increasingly frequent use.

After the oral appliance is covered with the formed foam, the cleaning composition is preferably washed off by rinsing with water, the oral appliance after application of the composition, without physical additive force. This enables the oral appliance to be cleaned simply and effectively.

The cleaning composition for an oral appliance of the present invention is preferably filled into a container equipped with a foam formation mechanism, and a foam is formed by injection or discharge from the container, from the viewpoint of sufficiently exerting the effect of the foam.

The container equipped with a foam formation mechanism is a container which can form a foam by injecting or discharging contents filled in the container to the outside of the container from an injection port or a discharge port via the foam formation mechanism. Examples of such a container equipped with a foam formation mechanism include trigger type injection containers and pump type dispenser containers.

The trigger type injection container has a container main body to be filled with contents, and a trigger and a sprayer which serve as the foam formation mechanism. The contents are injected from the injection port of the sprayer to the outside by pulling the trigger upon application so that a foam is formed. Such a trigger type injection container may be a direct pressure system or may be an accumulator system.

The pump type dispenser container has a container main body to be filled with contents, and a foam formation mechanism such as a tube, a nozzle, and a mesh inside thereof. The contents are discharged from the discharge port of the tube or the nozzle to the outside by partially pressing the container upon application so that a foam is formed.

In the case of cleaning an oral appliance using the cleaning composition for an oral appliance of the present invention, a trigger type injection container is preferably used among these containers from the viewpoint of sufficiently exerting the effect of the foam.

In the case of using a trigger type injection container, it is desirable to set an injection diameter in an injection distance of 20 cm to preferably 2 cm or longer, more preferably 3 cm or longer, and preferably 10 cm or shorter, more preferably 8 cm or shorter, from the viewpoint of sufficiently exerting the effect of the foam.

For the trigger type injection container, it is desirable to set an injection force of the cleaning composition as the contents in an injection distance of 20 cm to preferably 1 g·f or more, more preferably 3 g·f or more, and preferably 10 g·f or less, more preferably 8 g·f or less, from the viewpoint of validly forming a foam.

Thus, the cleaning composition for an oral appliance of the present invention, which can sufficiently remove the sliminess of an oral appliance by application for a short time without the need of particular physical additive force, can also reduce burdens on users such as elderly people and facilitate increasingly frequent use.

In relation to the embodiments mentioned above, the present invention further discloses the following cleaning composition for an oral appliance.

[1] A cleaning composition for an oral appliance, comprising the following components (A) and (B):

(A) a fatty acid having 10 or more and 20 or less carbon atoms or a salt thereof in an amount of 0.5 mass% or more and 20 mass% or less in terms of the fatty acid; and
(B) one or more surfactants selected from the group consisting of an anionic surfactant (b1), a nonionic surfactant (b2), and an amphoteric surfactant (b3), wherein

the component (b1) is one or more selected from the group consisting of an N-acyl amino acid, N-acyl taurine, and an α-olefinsulfonic acid, and salts thereof,
the component (b2) is one or more selected from the group consisting of polyoxyethylene hydrogenated castor oil, sorbitan fatty acid ester, fatty acid polyoxyethylene sorbitan ester, sucrose fatty acid ester, and alkyl glycoside,
the component (b3) is one or more selected from the group consisting of betaine acetate, cocamidoalkyl betaine, alkyl sulfobetaine, imidazolinium betaine, carboxybetaine, alkylimidazoline betaine salt, and alkylamine oxide, and

the composition has a pH at 25°C of 5.5 or higher and 14 or lower.

[2] The cleaning composition for an oral appliance according to [1], wherein the fatty acid as the component (A) or the fatty acid constituting the fatty acid salt as the component (A) has preferably 12 or more and 20 or less, more preferably 14 or more and 20 or less, further more preferably 16 or more and 20 or less carbon atoms, and optionally has a saturated or unsaturated linear or branched chain and more preferably has an unsaturated linear chain.

[3] The cleaning composition for an oral appliance according to [1] or [2], wherein a content of the component (A) is preferably 0.6 mass% or more, more preferably 0.7 mass% or more, further more preferably 0.8 mass% or more, and preferably 14 mass% or less, more preferably 5 mass% or less, further more preferably 3 mass% or less, in terms of the fatty acid.

[4] The cleaning composition for an oral appliance according to any one of [1] to [3], wherein the acyl group of the N-acyl amino acid or the N-acyl taurine as the component (b1) is derived from a fatty acid having a saturated or unsaturated linear or branched chain or a mixed fatty acid thereof, preferably a linear fatty acid or a mixed fatty acid of linear fatty acids, and is preferably an acyl group having 6 to 22 carbon atoms, more preferably an acyl group having 10 to 20 carbon atoms, further more preferably an acyl group having 12 to 18 carbon atoms.

[5] The cleaning composition for an oral appliance according to any one of [1] to [4], wherein the N-acyl amino acid as the component (b1) is preferably one or more selected from the group consisting of N-lauroyl glutamic acid, N-myristoyl glutamic acid, N-cocoyl glutamic acid, N-lauroyl aspartic acid, N-myristoyl aspartic acid, N-cocoyl aspartic acid, N-lauroyl glycine, N-myristoyl glycine, and N-cocoyl glycine, and the N-acyl taurine as the component (b1) is preferably one or more selected from the group consisting of cocoyl fatty acid taurine, cocoyl methyl taurine, N-caproyl methyl taurine, N-lauroyl taurine, N-lauroyl methyl taurine, N-myristoyl methyl taurine, N-palmitoyl methyl taurine, N-stearoyl methyl taurine, and N-oleoyl methyl taurine.

[6] The cleaning composition for an oral appliance according to any one of [1] to [5], wherein a content of the component (B) is preferably 0.05 mass% or more, more preferably 0.07 mass% or more, further more preferably 0.15 mass% or more, further more preferably 0.25 mass% or more, further more preferably 0.5 mass% or more, and preferably 5 mass% or less, more preferably 4 mass% or less, further more preferably 3 mass% or less.

[7] The cleaning composition for an oral appliance according to any one of [1] to [6], wherein the composition has a pH at 25°C of preferably 6 or higher, more preferably 6.5 or higher, further more preferably 7 or higher, even more preferably 7.5 or higher, even more preferably 8 or higher, even more preferably 8.5 or higher, even more preferably 8.8 or higher, even more preferably 9 or higher, even more preferably 9.5 or higher, and preferably 13 or lower, more preferably 12 or lower, further more preferably 11 or lower.

[8] The cleaning composition for an oral appliance according to any one of [1] to [7], wherein a mass ratio of the content of the component (B) to the content of the component (A), ((B)/(A)), is preferably 0.05 or more, more preferably 0.15 or more, further more preferably 0.25 or more, further more preferably 0.35 or more, further more preferably 0.5 or more, and preferably 2.5 or less, more preferably 2.2 or less, further more preferably 2 or less.

[9] The cleaning composition for an oral appliance according to any one of [1] to [8], preferably further comprising an anionic homopolymer or copolymer (C), wherein a mass average molecular weight of the component (C) is preferably 2,000 or higher, more preferably 5,000 or higher, further more preferably 8,000 or higher, even more preferably 10,000 or higher, and preferably 500,000 or lower, more preferably 350,000 or lower, further more preferably 200,000 or lower, even more preferably 100,000 or lower.

[10] The cleaning composition for an oral appliance according to [9], wherein a content of the component (C) is preferably 0.05 mass% or more, more preferably 0.07 mass% or more, further more preferably 0.2 mass% or more, even more preferably 0.3 mass% or more, and preferably 8 mass% or less, more preferably 7 mass% or less, further more preferably 6 mass% or less, even more preferably 5 mass% or less, even more preferably 4 mass% or less, even more preferably 2 mass% or less.

[11] The cleaning composition for an oral appliance according to [9] or [10], wherein a mass ratio of the content of the component (C) to the content of the component (A), ((C)/(A)), is preferably from 0.05 to 8, more preferably from 0.07 to 7, further more preferably from 0.1 to 6, even more preferably from 0.2 to 5, even more preferably from 0.3 to 4, even more preferably from 0.3 to 2.

[12] The cleaning composition for an oral appliance according to any one of [1] to [11], wherein a content of water (D) is preferably more than 50 mass%, more preferably 56 mass% or more, further more preferably 60 mass% or more, and preferably 99 mass% or less, more preferably 98.5 mass% or less, further more preferably 98 mass% or less.

[13] The cleaning composition for an oral appliance according to [12], wherein a mass ratio of the content of the component (D) to the content of the component (A), ((D)/(A)), is preferably from 50 to 99, more preferably from 55 to 98.5, further more preferably from 60 to 98.

[14] The cleaning composition for an oral appliance according to any one of [1] to [13], optionally further comprising a pH adjuster (E), wherein a content of the component (E) is preferably from 0.1 to 60 mass%, more preferably from 0.1 to 20 mass%, further more preferably from 0.15 to 10 mass%, even more preferably from 0.2 to 5 mass%.

[15] The cleaning composition for an oral appliance according to [14], wherein a mass ratio of the content of the component (E) to the content of the component (A), ((E)/(A)), is preferably from 0.1 to 60, more preferably from 0.1 to 20, further more preferably from 0.15 to 10, even more preferably from 0.2 to 5, even more preferably from 0.2 to 2.

[16] The cleaning composition for an oral appliance according to any one of [1] to [15], wherein the composition is applied in a liquid, foam, paste, gel, concentrated, powder, granule, or tablet form.

[17] A method for cleaning an oral appliance, the method comprising applying a cleaning composition for an oral appliance according to any one of [1] to [15] to the oral appliance, leaving it, and then rinsing the composition off.

[18] The method for cleaning an oral appliance according to [17], wherein an amount of time for which the cleaning composition for an oral appliance is applied to the oral appliance is from 1 minute to 30 minutes.

[19] The method for cleaning an oral appliance according to [17] or [18], the method comprising applying the cleaning composition for an oral appliance to the oral appliance, and then rinsing the composition with water.

[20] A method for using the cleaning composition for an oral appliance according to any one of [1] to [16], the method comprising applying the cleaning composition for an oral appliance to an oral appliance, leaving it, and then rinsing the composition off.

[21] The method for using the cleaning composition for an oral appliance according to [20], wherein an amount of time for which the cleaning composition for an oral appliance is applied to the oral appliance is from 1 minute to 30 minutes.

[22] The method for using the cleaning composition for an oral appliance according to [20] or [21], the method comprising applying the cleaning composition for an oral appliance to an oral appliance, and then rinsing the oral appliance with water.

[23'] Use of the composition according to any one of [1] to [16] as a composition for cleansing an oral appliance.

[24'] The use according to [23'], wherein the composition is applied to an oral appliance, left, and then rinsed off for use.

[25'] The use according to [23'] or [24'], wherein an amount of time for which the composition is applied to the oral appliance is from 1 minute to 30 minutes.

[26'] The use according to any one of [23'] to [25'], wherein after the composition is applied to an oral appliance, the oral appliance is rinsed with water.

Examples

Hereinafter, the present invention will be specifically described with reference to Examples. The content of each component in tables is indicated by mass%, unless otherwise specified.

[Examples 1 to 20 and Comparative Examples 1 to 5]

The components of each example described in Tables 1 to 8 were mixed to prepare each cleaning composition for an oral appliance. The obtained cleaning composition for an oral appliance was subjected to each evaluation in accordance with a method described below.

The results are shown in Tables 1 to 8.

The cleaning composition for an oral appliance obtained in Example 1 was filled into a container for foam discharge (a trigger type injection container set to an injection diameter of 5 cm and an injection force of 5 g·f in an injection distance of 20 cm), discharged in a foam from a discharge port, applied to used dentures, and left for 5 minutes, followed by rinsing with water to wash off the cleaning composition for an oral appliance. As a result, when the dentures cleaned with the cleaning composition for an oral appliance of the present invention were brought back into the oral cavity, the cleaning composition was able to sufficiently remove the denture plaque of the oral appliance by application for a short time, despite the absence of particular physical additive force such as brushing, and enabled non-slimy, high cleaning performance to be realized.

<<Evaluation of denture plaque removal effect>>

1) Production of denture plaque model

Glycerol stocks of *Streptococcus mutans* JCM5705 and *Candida albicans* JCM1542 were streaked onto an agar plate (Anaero Columbia Agar with RSB, Nippon Becton Dickinson Co., Ltd.), which was then stored, together with a $CO_2$ pack, in a plastic case and cultured at 37°C for 48 hours to 72 hours under anaerobic conditions. A single colony was picked up from the agar plate thus cultured, placed in a cell culture flask containing 20 mL of dispensed liquid medium A (3 mass% of SCD medium DAIGO (manufactured by Nihon Pharmaceutical Co., Ltd.), 0.5 mass% of Bacto Yeast Extract (manufactured by Becton, Dickinson and Company)), and stirred. Then, this flask was stored, together with a $CO_2$ pack, in a plastic case and cultured at 37°C for 24 hours under anaerobic conditions. This culture solution was diluted with liquid medium A so as to attain OD = 0.5. This diluted solution was diluted with liquid medium B (3 mass% of SCD medium DAIGO, 0.5 mass% of Bacto Yeast Extract, 1 mass% of D(+)-glucose (manufactured by FUJIFILM Wako Pure Chemical Corp.), 2 wt% of sucrose (manufactured by FUJIFILM Wako Pure Chemical Corp.)) so as to attain S. *mutans* OD = 0.01 and C. *albicans* OD = 0.00001. The diluted solution was further mixed with a sterilized aqueous solution of calcium chloride (manufactured by FUJIFILM Wako Pure Chemical Corp.) such that the final calcium ion concentration was 40 ppm to prepare a denture plaque model testing solution.

Subsequently, a denture substrate (manufactured by Kyoyukai Co., Ltd., material: polymethyl methacrylate resin, 1 cm) was placed in each well of a 24-well plate, and the prepared denture plaque model testing solution was added at 1 mL/well. Then, this plate was stored, together with a $CO_2$ pack, in a plastic case and cultured at 37°C for 16 hours under anaerobic conditions.

2) Evaluation of denture plaque removal effect

The denture plaque model testing solution in the plate was sucked out using a vacuum pump. Ion exchange water was added at 1 mL/well, and the plate was shaken for 5 minutes. Subsequently, the water was sucked out using a pump. The composition obtained in each of Examples and Comparative Examples was prepared at 1 mL/well in another 24-well plate. Subsequently, the denture substrate in which the denture plaque model was formed was dipped in the prepared solution and left for 5 minutes.

Then, each composition was sucked out, and ion exchange water was added at 1 mL/well, followed by shaking for 5 minutes. This operation was repeated three times. The shaking was performed under conditions of room temperature (25°C) and 550 rpm using a shaker (BioShake iQ (manufactured by WAKENBTECH Co., Ltd.)). Subsequently, the water was sucked out, and a 0.1 mass% crystal violet (CV) solution was added at 750 $\mu$L/well, followed by shaking for 15 minutes.

The CV staining solution was further sucked out using a pump, and ion exchange water was added at 1 mL/well, followed by shaking for 5 minutes. This operation was repeated twice. Subsequently, the water was sucked out using a pump, and ethanol was added at 500 $\mu$L/well, followed by pipetting. Then, the extract was diluted 10-fold with ion exchange water, and absorbance $OD_{595\,nm}$ was measured using a microplate recorder (wavelength-variable absorption microplate reader Sunrise Rainbow Thermo manufactured by Tecan Group Ltd.).

Absorbance $OD_{595\,nm}$ (initial value) measured when the denture substrate was only washed with ion exchange water without the use of the obtained composition was used as a reference, and a denture plaque removal rate (%) was calculated according to the equation (1) given below.

A larger value of the obtained denture plaque removal rate means a higher denture plaque removal effect.

$$\mathtt{Denture\ plaque\ removal\ rate\ (\%)\ =\ 100\ -\ \{OD_{595\ nm}}$$

$$\mathtt{measured\ when\ the\ obtained\ composition\ was\ used\ /\ OD_{595\ nm}}$$

$$\mathtt{measured\ when\ the\ denture\ substrate\ was\ only\ washed\ with}$$

$$\mathtt{ion\ exchange\ water\}\ \times\ 100\ ...\ (1)}$$

<<Evaluation of denture plaque formation suppressing effect>>

A denture substrate (manufactured by Kyoyukai Co., Ltd., material: polymethyl methacrylate resin, 1 cm) was placed in each well of a 24-well plate, and the composition obtained in each of Examples and Comparative Examples was prepared at 1 mL/well in another 24-well plate. After dipping for 5 minutes, the denture plaque model testing solution in the plate was sucked out using a vacuum pump, and the prepared denture plaque model testing solution in the plate was immediately added at 1 mL/well. Then, this plate was stored, together with a $CO_2$ pack, in a plastic case and cultured at 37°C for 16 hours under anaerobic conditions.

The denture plaque model testing solution in the plate was sucked out using a vacuum pump. Ion exchange water was added at 1 mL/well, and the plate was shaken for 5 minutes. Then, the rinse was sucked out, and ion exchange water was added at 1 mL/well, followed by shaking for 5 minutes. This operation was repeated three times. The shaking was performed under conditions of room temperature (25°C) and 550 rpm using a shaker (BioShake iQ (manufactured by WAKENBTECH Co., Ltd.)). Subsequently, the water was sucked out, and a 0.1 mass% crystal violet (CV) solution was added at 750 $\mu$L/well, followed by shaking for 15 minutes.

The CV staining solution was further sucked out using a pump, and ion exchange water was added at 1 mL/well, followed by shaking for 5 minutes. This operation was repeated twice. Subsequently, the water was sucked out using a pump, and ethanol was added at 500 μL/well, followed by pipetting. Then, the extract was diluted 10-fold with ion exchange water, and absorbance $OD_{595\,nm}$ was measured using a microplate recorder (wavelength-variable absorption microplate reader Sunrise Rainbow Thermo manufactured by Tecan Group Ltd.).

Absorbance $OD_{595\,nm}$ (initial value) measured when the denture substrate was only washed with ion exchange water without the use of the obtained composition was used as a reference, and a denture plaque formation suppression rate (%) was calculated according to the equation (2) given below.

A larger value of the obtained denture plaque formation suppression rate means a higher denture plaque formation suppressing effect.

$$\text{Denture plaque formulation suppression rate (\%)} = 100 - \{OD_{595\,nm} \text{ measured when the denture substrate was subjected to the pretreatment with the obtained composition} / OD_{595\,nm} \text{ measured when the denture substrate was subjected to only pretreatment with ion exchange water}\} \times 100 \ ... \ (2)$$

<<Appearance immediately after production>>

A glass bottle was filled with each obtained cleaning composition for an oral appliance, and the cleaning composition for an oral appliance as the contents was visually observed from the outside of the glass bottle and evaluated according to the following criteria.

A: The composition was not cloudy, was transparent, and was homogeneous without precipitates.
B: The composition was homogeneous without precipitates, though being somewhat cloudy.
C: The composition was homogeneous without precipitates, though being cloudy.
D: Separation or precipitates were confirmed.

<<Low-temperature storage stability>>

A glass bottle was filled with each obtained cleaning composition for an oral appliance, and storage was started at -5°C. After each lapse of 7 days, the cleaning composition for an oral appliance as the contents was visually observed from the outside of the glass bottle, and the number of days was counted until the cleaning composition for an oral appliance was solidified.

<<Suppressing effect on odor of fatty acid>>

One panelist (n = 1) who has evaluated odor on a regular basis brought the nose close to each cleaning composition for an oral appliance to smell the composition, and evaluated if its odor was good or poor. In evaluation criteria, a sample was given good when odor derived from fatty acid was masked, and other cases were given poor.

[Table 1]

| | | | (A) Example 1 | Example 2 | Example 3 | Example 4 | Example 5 |
|---|---|---|---|---|---|---|---|
| (A) | Oleic acid *1 | | 1 | 1 | 1 | 1 | 1 |
| (B) | Sodium N-myristoyl-L-glutamate (b1) *2 | | 0.1 | 0.4 | 0.1 | 0.2 | 0.4 |
| (C) | Acrylic acid/maleic acid copolymer *5 | | 0.8 | 0.8 | 0 | 0 | 0 |

(continued)

|  | | (A) Example 1 | Example 2 | Example 3 | Example 4 | Example 5 |
|---|---|---|---|---|---|---|
| (E) | Potassium hydroxide | 0.3 | 0.3 | 0.3 | 0.3 | 0.3 |
| | Sodium carbonate | 0.2 | 0.2 | 0.2 | 0.2 | 0.2 |
| | Sodium bicarbonate | 0.2 | 0.2 | 0.2 | 0.2 | 0.2 |
| | Fragrance | 0.2 | 0.2 | 0.2 | 0.2 | 0.2 |
| | Purified water | Balance | Balance | Balance | Balance | Balance |
| | Total | 100 | 100 | 100 | 100 | 100 |
| (D) | Water | 97.2 | 96.9 | 98.0 | 98.0 | 97.7 |
| | pH | 10 | 10 | 10 | 10 | 10 |
| | (B)/(A) | 0.1 | 0.4 | 0.1 | 0.2 | 0.4 |
| | (C)/(A) | 0.8 | 0.8 | 0.0 | 0.0 | 0.0 |
| | (D)/(A) | 97.2 | 96.9 | 98.0 | 98.0 | 97.7 |
| | (E)/(A) | 0.7 | 0.7 | 0.7 | 0.7 | 0.7 |
| Appearance immediately after production | | A | A | A | A | A |
| Low-temperature storage stability (the number of days required for solidification after start of storage at -5°C) | | 14 | 28 | >90 | >90 | >90 |
| *1: LUNAC O-V, manufactured by Kao Corp.<br>*2: AMISOFT MS, manufactured by Ajinomoto Healthy Supply Co., Inc.<br>*5: POIZ 520, mass average molecular weight = 21,000, acrylic acid/maleic acid = 70/30, manufactured by Kao Corp. | | | | | | |

[Table 2]

|  | | (A) Comparative Example 1 | Comparative Example 2 | Example 6 |
|---|---|---|---|---|
| (A) | Oleic acid *1 | 10 | 8 | 10 |
| (B) | Sodium N-myristoyl-L-glutamate (b1) *2 | 0 | 0 | 1 |
| (C) | Acrylic acid/maleic acid copolymer *5 | 0 | 0 | 0 |
| (E) | Potassium hydroxide | 2.9 | 2.3 | 2.9 |
| | Sodium carbonate | 0.0 | 0.0 | 0.0 |
| | Sodium bicarbonate | 0.0 | 0.0 | 0.0 |
| | Fragrance | 0.0 | 0.0 | 0.0 |
| | Purified water | Balance | Balance | Balance |
| | Total | 100 | 100 | 100 |
| (D) | Water | 87.1 | 89.7 | 86.1 |
| | pH | 13 | 13 | 13 |
| | (B)/(A) | 0.0 | 0.0 | 0.1 |
| | (D)/(A) | 8.7 | 11.2 | 8.6 |
| | (E)/(A) | 0.3 | 0.3 | 0.3 |

(continued)

|  | (A) Comparative Example 1 | Comparative Example 2 | Example 6 |
|---|---|---|---|
| Appearance immediately after production | D | A | A |
| Low-temperature storage stability (the number of days required for solidification after start of storage at -5°C) | - | 7 | 28 |
| *1, 2 and 5: Same as in Table 1 | | | |

[Table 3]

|  |  |  | Example 7 | Example 8 | Example 9 |
|---|---|---|---|---|---|
| (A) | Oleic acid *1 | | 1 | 1 | 1 |
| (B) | Sodium N-myristoyl-L-glutamate (b1) *2 | | 0.2 | 0 | 0.2 |
|  | Sodium lauroyl methyl taurine (b2) | | 0 | 0.3 | 0 |
|  | Sodium decyl sulfate | | 0 | 0 | 0.6 |
| (C) | Acrylic acid/maleic acid copolymer *5 | | 0.8 | 0.8 | 0.8 |
| (E) | Potassium hydroxide | | 0.3 | 0.3 | 0.3 |
|  | Sodium carbonate | | 0.2 | 0.2 | 0.2 |
|  | Sodium bicarbonate | | 0.2 | 0.2 | 0.2 |
|  | Fragrance | | 0.2 | 0.2 | 0.2 |
|  | Purified water | | Balance | Balance | Balance |
|  | Total | | 100 | 100 | 100 |
| (D) | Water | | 97.2 | 97.0 | 96.6 |
|  | pH | | 10 | 10 | 10 |
|  | (B)/(A) | | 0.2 | 0.3 | 0.8 |
|  | (C)/(A) | | 0.8 | 0.8 | 0.8 |
|  | (D)/(A) | | 97.2 | 97.0 | 96.6 |
|  | (E)/(A) | | 0.7 | 0.7 | 0.7 |
| Appearance immediately after production | | | A | A | A |
| Low-temperature storage stability (the number of days required for solidification after start of storage at -5°C) | | | 21 | 56 | >90 |
| Denture plaque removal rate(%) | | | 99 | 69 | 91 |
| *1, 2 and 5: Same as in Table 1 | | | | | |

[Table 4]

|  |  | Example 10 | Example 11 |
|---|---|---|---|
| (A) | Oleic acid *1 | 1 | 1 |
| (B) | Sodium N-myristoyl-L-glutamate (b1) *2 | 0.2 | 0.2 |
|  | Sodium N-cocoyl-L-glutamate (b1) *3 | 1 | 1 |
|  | Sodium lauryl sulfate | 0.1 | 0.3 |

(continued)

|  |  | Example 10 | Example 11 |
|---|---|---|---|
| (C) | Acrylic acid/maleic acid copolymer *5 | 0.8 | 0.8 |
| (E) | Potassium hydroxide | 0.3 | 0.3 |
|  | Propylene glycol | 3 | 3 |
|  | Glycerin | 5 | 5 |
|  | Phenoxyethanol | 0.8 | 0.8 |
|  | Sodium sulfite | 0.2 | 0.2 |
|  | Fragrance | 0.2 | 0.2 |
|  | Purified water | Balance | Balance |
|  | Total | 100 | 100 |
| (D) | Water | 87.6 | 87.4 |
|  | pH | 11 | 11 |
|  | (B)/(A) | 1.2 | 1.2 |
|  | (C)/(A) | 0.8 | 0.8 |
|  | (D)/(A) | 89.4 | 89.2 |
|  | (E)/(A) | 0.3 | 0.3 |
| Low-temperature storage stability (the number of days required for solidification after start of storage at -5°C) | | >63 | >63 |
| *1, 2 and 5: Same as in Table 1<br>*3: AMISOFT CS-22B, manufactured by Ajinomoto Healthy Supply Co., Inc. | | | |

[Table 5]

|  |  | Example 12 | Example 13 |
|---|---|---|---|
| (A) | Oleic acid *1 | 1 | 1 |
| (B) | Sodium N-myristoyl-L-glutamate (b1) *2 | 0.2 | 0.2 |
|  | Sodium N-cocoyl-L-glutamate (b1) *3 | 1 | 0 |
|  | Potassium cocoyl glycine (b1) *4 | 0 | 1 |
| (C) | Acrylic acid/maleic acid copolymer *5 | 0.8 | 0.8 |
| (E) | Potassium hydroxide | 0.3 | 0.3 |
|  | Sodium carbonate | 0.2 | 0.2 |
|  | Sodium bicarbonate | 0.2 | 0.2 |
|  | Fragrance | 0.2 | 0.2 |
|  | Purified water | Balance | Balance |
|  | Total | 100 | 100 |
| (D) | Water | 96.2 | 96.2 |
|  | pH | 10 | 10 |
|  | (B)/(A) | 1.2 | 1.2 |
|  | (C)/(A) | 0.8 | 0.8 |
|  | (D)/(A) | 96.2 | 96.2 |

(continued)

| | | Example 12 | Example 13 |
|---|---|---|---|
| | (E)/(A) | 0.7 | 0.7 |
| Appearance immediately after production | | A | A |
| Low-temperature storage stability (the number of days required for solidification after start of storage at -5°C) | | >90 | >90 |
| Denture plaque removal rate(%) | | 97 | 80 |
| Suppressing effect on odor of fatty acid | | good | good |

*1, 2 and 5: Same as in Table 1
*3: Same as in Table 4
*4: AMILITE GCK-12K, manufactured by Ajinomoto Healthy Supply Co., Inc.

[Table 6]

| | | Comparative Example 3 | Comparative Example 4 | Comparative Example 5 |
|---|---|---|---|---|
| (A) | Oleic acid *1 | 1 | 1 | 1 |
| | Sodium dodecyl sulfate | 0 | 0.5 | 0 |
| (C) | Acrylic acid/maleic acid copolymer *5 | 0.8 | 0.8 | 0 |
| (E) | Potassium hydroxide | 0.3 | 0.3 | 0 |
| | Sodium carbonate | 0.2 | 0.2 | 0 |
| | Sodium bicarbonate | 0.2 | 0.2 | 0 |
| | Fragrance | 0.2 | 0.2 | 0.2 |
| | Purified water | Balance | Balance | Balance |
| | Total | 100 | 100 | 100 |
| (D) | Water | 97.3 | 96.8 | 98.8 |
| | pH | 10 | 10 | 4.8 |
| | (B)/(A) | 0 | 0 | 0 |
| | (C)/(A) | 0.8 | 0.8 | 0 |
| | (D)/(A) | 97.3 | 96.8 | 98.8 |
| | (E)/(A) | 0.7 | 0.7 | 0.7 |
| Appearance immediately after production | | A | A | D |
| Low-temperature storage stability (the number of days required for solidification after start of storage at -5°C) | | 7 | 7 | |
| Suppressing effect on odor of fatty acid | | poor | | |

*1 and 5: Same as in Table 1

[Table 7]

| | | Example 14 |
|---|---|---|
| (A) | Potassium oleate *6 | 1 |

(continued)

|  |  | | Example 14 |
|---|---|---|---|
| (B) | | Sodium N-myristoyl-L-glutamate (b1) *2 | 0.2 |
| | | Sodium N-cocoyl-L-glutamate(b1) *3 | 1 |
| (C) | | Acrylic acid/maleic acid copolymer *5 | 0.8 |
| (E) | | Potassium hydroxide | 0.1 |
| | | Potassium carbonate | 0.3 |
| | | Potassium bicarbonate | 0.2 |
| | | Propylene glycol | 3 |
| | | Glycerin | 5 |
| | | Purified water | Balance |
| | | Total | 100 |
| (D) | | Water | 88.4 |
| | | pH | 11 |
| | | (B)/(A) | 0.2 |
| | | (C)/(A) | 0.8 |
| | | (D)/(A) | 88.4 |
| | | (E)/(A) | 0.7 |
| Denture plaque formation suppression rate(%) | | | 59 |
| *2, 3 and 5: Same as in Table 1<br>*6: Manufactured by Tokyo Chemical Industry Co., Ltd. | | | |

[Table 8]

|  |  |  | Example 15 | Example 16 | Example 17 | Example 18 | Example 19 | Example 20 |
|---|---|---|---|---|---|---|---|---|
| (A) | | Potassium oleate *6 | 1 | 1 | 1 | 1 | 1 | 1 |
| (B) | | Sodium α-olefinsulfonate (b1) *7 | 1 | 4 | | | | |
| | | Cocamidopropyl betaine (b3) *8 | | | 1 | 4 | | |
| | | Lauryl dimethylamine oxide (b3) *9 | | | | | 1 | |
| | | Polyoxyethylene hydrogenated castor oil (b2) *10 | | | | | | 1 |
| (C) | | Acrylic acid/maleic acid copolymer *5 | 1 | 1 | 1 | 1 | 1 | 1 |
| (E) | | Sodium carbonate | 0.2 | 0.2 | 0.2 | 0.2 | 0.2 | 0.2 |
| | | Sodium bicarbonate | 0.2 | 0.2 | 0.2 | 0.2 | 0.2 | 0.2 |
| | | Fragrance | 0.2 | 0.2 | 0.2 | 0.2 | 0.2 | 0.2 |
| | | Purified water | Balance | Balance | Balance | Balance | Balance | Balance |
| | | Total | 100 | 100 | 100 | 100 | 100 | 100 |

(continued)

|  |  | Example 15 | Example 16 | Example 17 | Example 18 | Example 19 | Example 20 |
|---|---|---|---|---|---|---|---|
| (D) | Water | 96.62 | 93.62 | 96.62 | 93.62 | 96.62 | 96.62 |
|  | pH | 10 | 10 | 10 | 10 | 10 | 10 |
|  | (B)/(A) | 1 | 4 | 1 | 4 | 1 | 1 |
|  | (C)/(A) | 1 | 1 | 1 | 1 | 1 | 1 |
|  | (D)/(A) | 96.6 | 93.6 | 96.6 | 93.6 | 96.6 | 96.6 |
|  | (E)/(A) | 0.4 | 0.4 | 0.4 | 0.4 | 0.4 | 0.4 |
| Low-temperature storage stability (the number of days required for solidification after start of storage at -5°C) | | 21 | >28 | 14 | >28 | >28 | 14 |

*5: Same as in Table 1
*6: Same as in Table 7
*7: LIPOLAN PJ-400CJ, manufactured by Lion Specialty Chemicals Co., Ltd.
*8: TEGO BETAIN CK KB 5 S, manufactured by Evonik Nutrition & Care GmbH
*9: Anhitol 20N, manufactured by Kao Corp.
*10: EMANON CH-40, manufactured by Kao Corp.

Formulation examples of the cleaning composition for an oral appliance of the present invention will be described below.

The "molecular weight" in the formulation examples means a "mass average molecular weight".

[Formulation Example 1]

| Sodium oleate | 1.0 |
|---|---|
| Sorbitan stearate | 1.0 |
| Propylene glycol | 5.0 |
| Glycerin | 5.0 |
| Phenoxyethanol | 0.8 |
| Sodium carbonate | 0.2 |
| Sodium bicarbonate | 0.2 |
| Fragrance | 0.1 |
| Purified water | Balance |
| Total | 100.0% |

[Formulation Example 2]

| Sodium laurate | 1.0 |
|---|---|
| Polyoxyethylene sorbitan monostearate | 0.5 |
| Propylene glycol | 5.0 |
| Glycerin | 5.0 |
| Phenoxyethanol | 0.8 |
| Sodium carbonate | 0.2 |
| Sodium bicarbonate | 0.2 |
| Fragrance | 0.1 |

(continued)

| Purified water | Balance |
|---|---|
| Total | 100.0% |

[Formulation Example 3]

| Sodium decanoate | 1.0 |
|---|---|
| Sucrose fatty acid ester | 1.0 |
| Propylene glycol | 5.0 |
| Glycerin | 5.0 |
| Phenoxyethanol | 0.8 |
| Isopropyl methyl phenol | 0.1 |
| Fragrance | 0.1 |
| Purified water | Balance |
| Total | 100.0% |

[Formulation Example 4]

| Sodium Linoleate | 1.5 |
|---|---|
| Alkyl glycoside | 0.5 |
| Glycerin | 10.0 |
| Sorbitol | 5.0 |
| Sodium benzoate | 1.0 |
| Phenoxyethanol | 0.8 |
| Gantrez S-97 BF*11 (PVM/MA copolymer) | 0.1 |
| Fragrance | 0.1 |
| Purified water | Balance |
| Total | 100.0% |
| *11: manufactured by Ashland Japan Ltd. (Gantrez(R)) | |

[Formulation Example 5]

| Acrylic acid/maleic acid copolymer (molecular weight: 21,000) | 4.0 |
|---|---|
| Potassium oleate | 5.0 |
| Sodium N-cocoyl-L-glutamate | 10.0 |
| Sodium carbonate | 10.0 |
| Sodium bicarbonate | 15.0 |
| Citric acid | 15.0 |
| Polyethylene glycol | 2.0 |
| Sodium percarbonate | 10.0 |
| Tetraacetylethylenediamine | 5.0 |
| Sodium benzoate | 2.0 |
| Protease | 1.0 |
| Fragrance | 0.5 |
| Sodium sulfate | Balance |

(continued)

| | |
|---|---|
| Total | 100.0% |

The components were well mixed, and 10 g thereof was compressed in a tableting machine. One tablet thus obtained was dissolved in 90 mL of water for use.

[Formulation Example 6]

| | |
|---|---|
| Sodium laurate | 5.0 |
| Polyoxyethylene hydrogenated castor oil | 10.0 |
| Sodium carbonate | 10.0 |
| Sodium bicarbonate | 15.0 |
| Citric acid | 15.0 |
| Polyethylene glycol | 2.0 |
| Sodium percarbonate | 10.0 |
| Tetraacetylethylenediamine | 5.0 |
| Sodium benzoate | 2.0 |
| Protease | 1.0 |
| Fragrance | 0.5 |
| Sodium sulfate | Balance |
| Total | 100.0% |

The components were well mixed, and 10 g thereof was compressed in a tableting machine. One tablet thus obtained was dissolved in 90 mL of water for use.

[Formulation Example 7]

| | |
|---|---|
| Acrylic acid/maleic acid copolymer (molecular weight:70,000) | 4.0 |
| Sodium linoleate | 5.0 |
| Cocamidopropyl betaine | 10.0 |
| Sodium carbonate | 10.0 |
| Sodium bicarbonate | 15.0 |
| Citric acid | 15.0 |
| Polyethylene glycol | 2.0 |
| Sodium percarbonate | 10.0 |
| Tetraacetylethylenediamine | 5.0 |
| Sodium benzoate | 2.0 |
| Protease | 1.0 |
| Fragrance | 0.5 |
| Sodium sulfate | Balance |
| Total | 100.0% |

The components were well mixed, and 10 g thereof was compressed in a tableting machine. One tablet thus obtained was dissolved in 90 mL of water for use.

[Formulation Example 8]

| | |
|---|---|
| Potassium oleate | 5.0 |
| Sodium $\alpha$-olefinsulfonate | 10.0 |
| Sodium carbonate | 10.0 |
| Sodium bicarbonate | 15.0 |
| Citric acid | 15.0 |
| Polyethylene glycol | 2.0 |
| Sodium percarbonate | 10.0 |
| Tetraacetylethylenediamine | 5.0 |
| Sodium benzoate | 2.0 |
| Protease | 1.0 |
| Fragrance | 0.5 |
| Sodium sulfate | Balance |
| Total | 100.0% |

The components were well mixed, and 10 g thereof was compressed in a tableting machine. One tablet thus obtained was dissolved in 90 mL of water for use.

[Formulation Example 9]

| | |
|---|---|
| Acrylic acid/maleic acid copolymer (molecular weight:70,000) | 4.0 |
| Sodium linoleate | 5.0 |
| Cocamidopropyl betaine | 10.0 |
| Sodium carbonate | 10.0 |
| Sodium bicarbonate | 15.0 |
| Citric acid | 15.0 |
| Polyethylene glycol | 2.0 |
| Sodium percarbonate | 10.0 |
| Tetraacetylethylenediamine | 5.0 |
| Sodium benzoate | 2.0 |
| Protease | 1.0 |
| Fragrance | 0.5 |
| Sodium sulfate | Balance |
| Total | 100.0% |

The components were well mixed, and 10 g thereof was compressed in a tableting machine. One tablet thus obtained was dissolved in 90 mL of water for use.

[Formulation Example 10]

| | |
|---|---|
| Acrylic acid/maleic acid copolymer (molecular weight:21,000) | 4.0 |
| Potassium oleate | 5.0 |
| Sodium N-acyl taurine | 10.0 |
| Sodium carbonate | 14.0 |
| Sodium bicarbonate | 21.0 |
| Citric acid | 21.0 |
| Polyethylene glycol | 2.0 |
| Sodium percarbonate | 10.0 |

(continued)

| Tetraacetylethylenediamine | 5.0 |
|---|---|
| Sodium benzoate | 2.0 |
| Protease | 1.0 |
| Fragrance | 0.5 |
| Sodium sulfate | Balance |
| Total | 100.0% |

The components were well mixed to form powder. 10 g thereof was dissolved in 90 mL of water for use.

## Claims

1. A cleaning composition for an oral appliance, comprising the following components (A) and (B):

   (A) a fatty acid having 10 or more and 20 or less carbon atoms or a salt thereof in an amount of 0.5 mass% or more and 20 mass% or less in terms of the fatty acid; and
   (B) one or more surfactants selected from the group consisting of an anionic surfactant (b1), a nonionic surfactant (b2), and an amphoteric surfactant (b3), wherein

   the component (b1) is one or more selected from the group consisting of an N-acyl amino acid, N-acyl taurine, and an $\alpha$-olefinsulfonic acid, and salts thereof,
   the component (b2) is one or more selected from the group consisting of polyoxyethylene hydrogenated castor oil, sorbitan fatty acid ester, fatty acid polyoxyethylene sorbitan ester, sucrose fatty acid ester, and alkyl glycoside,
   the component (b3) is one or more selected from the group consisting of betaine acetate, cocamidoalkyl betaine, alkyl sulfobetaine, imidazolinium betaine, carboxybetaine, alkylimidazoline betaine salt, and alkylamine oxide, and
   the composition has a pH at 25°C of 5.5 or higher and 14 or lower.

2. The cleaning composition for an oral appliance according to claim 1, wherein a mass ratio of a content of the component (B) to a content of the component (A), ((B)/(A)), is from 0.05 to 5.

3. The cleaning composition for an oral appliance according to claim 1 or 2, wherein the component (B) is the component (b1), and an acyl group of the component (b1) is derived from coconut oil.

4. The cleaning composition for an oral appliance according to any one of claims 1 to 3, further comprising an anionic homopolymer or copolymer (C).

5. The cleaning composition for an oral appliance according to claim 4, wherein a mass average molecular weight of the component (C) is from 2,000 to 500,000.

6. The cleaning composition for an oral appliance according to any one of claims 1 to 5, further comprising more than 50 mass% and 99 mass% or less of water (D).

| | | INTERNATIONAL SEARCH REPORT | International application No. |
|---|---|---|---|
| | | | PCT/JP2021/002626 |

**A. CLASSIFICATION OF SUBJECT MATTER**
Int. Cl. C11D17/08(2006.01)i, A61Q11/00(2006.01)i, A61Q11/02(2006.01)i, C11D1/04(2006.01)i, C11D1/10(2006.01)i, C11D1/14(2006.01)i, C11D1/18(2006.01)i, C11D1/68(2006.01)i, C11D1/74(2006.01)i, C11D1/75(2006.01)i, C11D1/90(2006.01)i, C11D 1/92(2006.01)i, C11D3/37(2006.01)i, A61K8/36(2006.01)i; A61K8/37(2006.01)i, A61K8/41(2006.01)i, A61K8/44(2006.01)i, A61K8/46(2006.01)i, A61K8/49(2006.01)i, A61K8/60(2006.01)i
FI: A61K8/36, A61K8/44, A61Q11/02, C11D1/04, C11D1/10, C11D3/37, C11D1/18, A61Q11/00, A61K8/46, A61K8/37, A61K8/60, A61K8/49, A61K8/41, C11D1/14, C11D1/74, C11D1/68, C11D1/90, C11D1/92, C11D1/75, C11D17/08

According to International Patent Classification (IPC) or to both national classification and IPC

**B. FIELDS SEARCHED**

Minimum documentation searched (classification system followed by classification symbols)
Int. Cl. A61K8/00-8/99, A61Q1/00-90/00, C11D1/00-19/00

Documentation searched other than minimum documentation to the extent that such documents are included in the fields searched
Published examined utility model applications of Japan      1922-1996
Published unexamined utility model applications of Japan    1971-2021
Registered utility model specifications of Japan            1996-2021
Published registered utility model applications of Japan    1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2012-153863 A (OKUDA, Yoshimasa) 16 August 2012, claims, paragraphs [0013], [0044]-[0046], [0066] | 1-2, 6 |
| Y | JP 2011-195496 A (TECHNOMINING INC.) 06October 2011, claims, paragraphs [0005], [0016]-[0017], [0020], [0028]-[0030] | 1-6 |
| Y | JP 2012-126758 A (KAO CORP.) 05 July 2012, paragraph [0014]-[0019], [0030]-[0041] | 1-6 |
| Y | JP 2000-247851 A (LION CORP.) 12 September 2000, paragraph [0005], [0009]-[0011], [0035]-[0039] | 4-6 |

| ☐ Further documents are listed in the continuation of Box C. | ☒ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 16.03.2021 | 30.03.2021 |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

| Patent Documents referred to in the Report | Publication Date | Patent Family | Publication Date |
|---|---|---|---|
| International application No. | | | PCT/JP2021/002626 |
| JP 2012-153863 A | 16.08.2012 | (Family: none) | |
| JP 2011-195496 A | 06.10.2011 | (Family: none) | |
| JP 2012-126758 A | 05.07.2012 | (Family: none) | |
| JP 2000-247851 A | 12.09.2000 | (Family: none) | |

Form PCT/ISA/210 (patent family annex) (January 2015)

**EP 4 166 639 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010280588 A

- JP 2011195496 A